# EUROPEAN PATENT SPECIFICATION

(11) **EP 0 329 794 B1**
(45) Date of publication and mention of the grant of the patent: **30.03.1994**
(21) Application number: 88907385.4
(22) Date of filing: 30.08.1988
(51) Int. Cl.: G01N 33/53, G01N 33/531, G01N 33/577, G01N 33/543

(54) **METHOD FOR ASSAYING HUMAN LUNG SURFACE ACTIVE SUBSTANCE**
VERFAHREN ZUM TESTEN EINER WIRKSAMEN SUBSTANZ DIE SICH AUF DER OBERFLÄCHE MENSCHLICHER LUNGEN BEFINDET
PROCEDE D'ANALYSE D'UNE SUBSTANCE ACTIVE SE TROUVANT A LA SURFACE DES POUMONS CHEZ L'HOMME

(30) Priority: 01.09.1987 JP 216355/87
(43) Date of publication of application: 30.08.1989
(73) Proprietor: TEIJIN LIMITED, Osaka-shi Osaka 541 (JP)
(72) Inventor: HOSODA, Kenji, Saitama 350 (JP); HONDA, Hitomi, Tokyo 191 (JP); KUBOTA, Takaaki, Tokyo 191 (JP); SUZUKI, Hideaki, Koganei-shi Tokyo 184 (JP)
(74) Representative: Votier, Sidney David
(86) International application number: JP8800861
(87) International publication number: WO8902075

(56) References cited:
- EP-A- 0 224 590
- JP-A- 5 997 057
- JP-A- 6 264 956
- JP-A-58 187 862
- JP-A-61 275 654
- JP-A-61 277 699
- JP-A-61 277 700
- JOURNAL OF IMMUNOLOGY, vol. 119, no. 5, November 1977, pages 1645-1651, The Williams & Wilkins Co., US; L.F. QUALTIERE et al.: "Effects of ionic and nonionic detergents on antigen-antibody reactions"
- BIOLOGICAL ABSTRACTS, vol. 88, no. 1, 1989, abstract no. 2165, Biological Abstracts, Inc., Philadelphia, PA, US; H. SHIMIZU et al.: "Improved immunoassay for the determination of surfactant protein A(SP-A) in human ammiotic fluid", & TOHOKU J. EXP. MED. 157(3): 269-278. 1989

## Description

The present invention relates to a method for quantitating the human pulmonary surfactant with the use of monoclonal antibodies against the human pulmonary surfactant apoproteins.

In the alveoli of the lung of an animal, there exists a physiologically active substance which mainly comprises phospholipid and is called a pulmonary surfactant. While covering the inner walls of the alveoli, this substance displays the activity to protect the alveolar epithelium and has an important physiological function for the animal to maintain its respiratory functions. More particularly, it is said that the pulmonary surfactant exerts a specific surface action to cause changes in the surface tension of the inner surfaces of the alveoli in response to expiration and inspiration, thus contributing to the maintenance of stability among the alveoli and manifestation of the antiatelectasis as well. The deficiency of such a pulmonary surfactant can lead to the collapse of the alveoli making them unable to maintain stabilized ventilation and thus causing the idiopathic respiratory distress syndrome (IRDS) which is sometimes seen with newborns.

Means can be adopted to prevent a newborn from being born with such a syndrome: when the result of the determination of the pulmonary surfactant content in the amniotic fluid, which is correlated to the growth of the fetal lungs, shows that the fetus is going to be born with immature lungs, it is possible to exercise such intrauterine therapy for the fetus as augmentation of secretion of the pulmonary surfactant by administration of steroid.

As regards the methods of determining or estimating the content of pulmonary surfactant in the amniotic fluid, several methods have hitherto been proposed. For example, there is a method in which the L/C ratio (ratio between lecithin and sphingomyelin) in the amniotic fluid is determined and another method in which the amount of dipalmitoyl phosphatidyl choline (DPPC) in the amniotic fluid is measured as the marker of the pulmonary surfactant; however, the former has the disadvantage a of presenting a low correlation with IRDS since the method does not determine the phospholipid content which is the main component of the pulmonary surfactant, and the latter method suffers from having a low sensitivity.

In adults, deficiency of the pulmonary surfactant causes symptoms such as adult respiratory distress syndrome (ARDS). The pulmonary surfactant in lung washing, for instance, DPPC or phosphatidyl glycerol (PG) as a marker for the surfactant, is determined for diagnosis of pulmonary diseases. The determination, however, encounters the problem of unsatisfactory sensitivity.

In the meantime, the lung washing is physicochemically measured, for example, to determine its surface tension. Sputum from patients is also used to make diagnosis, for example, by examining its viscosity and color tone or measuring fucose, reflecting the amount of tracheal mucus in the sputum. Sputum can be collected more easily than lung washing and this means the former is superior to the latter as a sample for measurement.

Incidentally, about 90% of the pulmonary surfactant is lipids such as phospholipid and neutral lipid and about 10% is protein. They exist as a complex of lipid and protein, i.e. as the lipoprotein. The removal of lipids from the pulmonary surfactant gives water-insoluble protein, which is called apoprotein (LSP) mainly composed of protein with a molecular weight of about 36,000 (36K). Since protein has a greater specificity than phospholipid and can be detected at higher sensitivity, studies have been made as to the use of protein as the marker of the pulmonary surfactant and immunological quantitation using polyclonal antibodies has also been attempted. However, problems are also found with the method, in which polyclonal antibodies are used, in that the determination procedure takes a long time and that the sensitivity is insufficient.

To cope with such problems, the present inventors have already produced monoclonal antibodies against the apoproteins constituting the pulmonary surfactant and proposed a method for quantitating the pulmonary surfactant by use of the monoclonal antibodies [Japanese Patent Laid-open Specification No. 62-64956 (1987)]. Difference in reactivity has been found, however, between the standard sample of apoprotein and the pulmonary surfactant in the test samples such as amniotic fluid or sputum, when the immunological measurement was made, as stated above. This is because the pulmonary surfactant in the samples is composed of apoprotein and 10 times its amount of phospholipid (in the case of sputum, tracheal mucus is additionally included) and the immunoreactions are inhibited by these phospholipids and tracheal mucus. In order to overcome the problems, the immunological reaction must be continued for a long time and it is thus inconvenient for the diagnosis of acute diseases such as IRDS or ARDS.

The present inventors have therefore studied the conditions for immunological measurement with two kinds of monoclonal antibodies and polyclonal antibodies which are specific to apoprotein, described in Japanese Patent Specification Laid-open No. 62-64956. As a result, they found that the apoprotein and the lipoprotein in the samples reached the same equivalent point in the immunological measurement in a short time, in other words the apoprotein in lipoproteins can be correctly captured, by a suitable selection of surfactant to be added to the buffer solution for the immunoreactions.

The present invention accordingly provides an assay for quantitating human pulmonary surfactant by determining the amount of apoprotein in a sample of human pulmonary surfactant by the immunological method based on antigen-antibody reactions wherein the immunoreactions are conducted in a buffer solution containing a combination of nonionic surfactant and anionic surfactant, the ratio of said nonionic surfactant to said anionic surfactant being such that the antigen-antibody reaction is not inhibited by protein denaturation of the antigen protein and that the nonionic surfactant does not mask the effect of the anionic surfactant to disperse or loosen phospholipids and respiratory mucus surrounding the apoprotein.

In the accompanying drawings,
Figure 1 shows the observed values of pulmonary surfactants (lipoproteins) in an amniotic fluid sample, when the concentration of SDS is varied in the buffer solution (2% Triton) for dilution of amniotic fluid samples (the immunoreactions were conducted at 37°C for 1 hour).
Figure 2 shows the observed values of pulmonary surfactants (lipoproteins) in an amniotic fluid sample, when the concentration of Triton® is varied in the buffer solution (1% SDS) for dilution of amniotic fluid samples (the immunoreactions were conducted at 37°C for 1 hour).
Figure 3 shows the observed values of pulmonary surfactants (lipoproteins) in an amniotic fluid sample, when the concentration of SDS is varied in the buffer solution (2% Triton) for dilution of amniotic fluid samples (the immunoreactions were conducted at 37°C for 30 minutes).
Figure 4 gives change of the observed values of pulmonary surfactant (lipoproteins) in amniotic fluid with the passage of time during the immunoreactions.
Figure 5 gives the observed values of pulmonary surfactant in a sputum sample, when the concentrations of SDS and Triton are varied.
Figure 6 gives the amounts of pulmonary surfactant (LSP) from patients with chronic respiratory diseases and bronchoalveolar epithelial cancer and LSP/fucose ratios.

It has been made possible to carry out solid-phase immunoreactions in a short time according to the present invention. The reasons may be cited as follows: when only nonionic surfactant is employed, there is no adverse effect on the immunoreaction, but the surface-activating effect is unsatisfactory. Similarly, the use of only anionic surfactant results in powerful surface-activating effect, but in inhibition of the antigen-antibody reactions, as the protein of the antigen is denatured. The combination of anionic and nonionic surfactants manifests, however, the merits of both surfactants, powerful surface activation due to anionic surfactant and protein protection due to nonionic surfactant, to make it possible to shorten the time of immunoreactions.

In the present invention, samples mean human amniotic fluid, sputum, lung washing and the like.

There is no particular limit as to the immunological method according to the present invention, and either polyclonal or monoclonal antibodies against the apoprotein of pulmonary surfactant can be used as an antibody in the antigen-antibody reactions. These antibodies can be produced by known methods.

As the surface active agent which is used in the present invention, are cited nonionic agents such as polyoxyethylene alkyl ethers, polyoxyethylene fatty acid (mono)esters, polyoxyethylene alkyl thioethers and anionic agents such as higher alcohol sulfate ester, alkylsulfonate salts, alkylbenzenesulfonate salts, dinaphthylmethanedisulfonate salts, alkylsulfosuccinate salts, polyoxyethylene alkyl ether sulfate ester, polyoxyethylene alkylphenol sulfate ester or the like. A polyoxyethylene alkylphenol ether, for example, Triton® (the trade name of a product manufactured by Rohm & Haas Co.) is preferred as the nonionic surfactant, while an alkylsulfonate salt, for example sodium dodecylsulfate (SDS), is preferred as the anionic one. The concentrations are preferably about 0.25 to 4% by weight in the former and about 0.2 to 3% by weight in the latter, and the ratio of the concentrations on a weight basis is particularly preferred to be 1.0 to 4.0. Less than 1.0 of Triton/SDS concentration ratio shows a tendency to inhibit the antigen-antibody reactions by protein denaturation of the antibodies, as SDS with powerful surface-activating effect predominates. At the same time, a ratio of over 4.0 reveals a prominent protein-protecting effect by the nonionic surfactant to mask the effect of the anionic surfactant to disperse or loosen the phospholipids and respiratory mucus surrounding the apoprotein.

According to the present invention, the primary antibody is preferably immobilized on a support and the immobilisation can be done by a known method. The support is solid and balls, beads, gears and microplates from polystyrene, polyethylene, polyacrylates, Teflon, polyacetal or the like are preferably used as the support.

No limit is placed upon the method and procedure of labeling and the method and procedure of detecting. Any known methods and procedures such as determination by the secondary reactions with anti-immunoglobulin antibody labeled with a radioactive substance, enzyme or fluorescent material, or with Staphylococcus protein A may be adopted. As the labeling agents, such enzymes as horseradish peroxidase, β-D-galactosidase or alkaline phosphatase are used in the enzyme immunoassay (EIA), ²⁵I and ³H in the radioimmunoassay (RIA), and fluorescein-isothiocyanate in the fluorescence immunoassay (FIA) in general; however, other labeling agents may also be used so far as their activity is assayable.

When the labeling agent is an enzyme, a substrate is used for assaying its activity. As the substrate, 2,2'-azinodi-[3-ethylbenzthiazoline sulfonic acid]ammonium acid (ABTS)-H₂O₂, 5-aminosalicylic acid-H₂O₂, o-phenylenediamine-H₂O₂ and 4-aminoantipyrine-H₂O₂ may be used as the substrate for horseradish peroxidase, and fluorescein-di-(β-D-galactopyranoside) o-nitrophenol-β-D-galactopyranoside for β-D-galactosidase. In the assays, other reagents such as solubilizer, detergents and reaction terminator are additionally used.

The method provided by the present invention is expected to offer a useful method for quantitating the apoproteins contained in the pulmonary surfactant in samples with ease in a short time for the diagnosis of diseases which need attention such as IRDS or ARDS.

The following examples illustrate the present invention.
% in the examples stands for % by weight.

### Example 1

(1) After polystyrene beads were thoroughly washed, they were placed in a PBS (pH 7.4) solution containing monoclonal antibody PC 6 at a concentration of 20 µg/ml and left at 4°C overnight. Thereafter, the beads were washed in a PBS solution and then left standing in 0.5% bovine serum albumin (BSA) aqueous solution at 4°C overnight for postcoating treatment to give beads of insolubilized monoclonal antibodies.
(2) Preparation of horseradish peroxidase-labeled monoclonal antibodies
   50 ml of dimethylformamide solution containing N-(m-maleimide benzoic acid)-N-succinimide ester (MBS) (10 mg/ml) was added to a PBS solution containing monoclonal antibody PE10 (1.0 mg/ml) and the reaction was conducted at 25°C for 30 minutes. The reaction mixture was then subjected to gel filtration with 0.1M phosphate buffer (pH 6.0) by use of a column filled with Sephadex G-25 to separate maleimidic monoclonal antibodies from unreacted MBS.
   In addition to the above procedure, an ethanol solution of N-succinimidyl-3-(3-pyridylthio)propionate (SPOP) (10 mg/ml) was added to a PBS solution containing horseradish peroxidase (HRP) (1.0 mg/ml) and the mixture was allowed to react at 25°C for 30 minutes. Then, the reaction mixture was purified by filtration with 0.01M acetate buffer (pH 4.5) by use of a Sephadex® G25 column and the fractions containing dithiopyridyl group substituted HRP were collected and concentrated approximately to about one-tenth of the original volume in a collodion bag on an ice bath. Then, 1 ml of 0.1M acetate buffer (pH 4.5) containing 0.85% NaCl and 0.1 M dithiothreitol was added thereto and the mixture was stirred at 25°C for 30 minutes to reduce the pyridyldisulfide groups introduced into HRP molecules. The reaction mixture was then subjected to gel filtration by Sephadex G-25 column to obtain fractions containing mercaptosuccinyl HRP.
   The maleimidic monoclonal antibodies and the mercaptosuccinyl HRP obtained in the above were mixed and concentrated to 4 mg of protein concentration in a collodion bag on the ice bath. The concentrate was left standing at 4°C overnight and filtered on a column of ultrogel AcA44 to obtain HRP-labeled monoclonal antibodies.
(3) Quantitation of pulmonary surfactant in amniotic fluid by change of SDS concentration (immunoreaction temperature: 37°C)
   As a buffer for diluting the amniotic fluid samples, mixtures of 2.0% Triton X-100 with SDS solutions of prescribed concentrations (0, 0.25, 0.5, 0.75, 1.0, 2.0, 3.0 and 4.0%) were used. These mixed solutions containing the amniotic fluid samples and a diluted solution containing monoclonal antibodies, PE 10 labeled with HRP, 200 µl each, were added to balls bearing monoclonal antibody PC6 in test tubes to effect immunoreactions at 37°C for 1 hour. The solutions in the test tubes were sucked off, the remaining balls were washed with physiological saline solution. Then, a 3/7 (V/V) mixture of a methanol solution containing 1% 3,3'5,5'-tetramethylbenzidine/1M phosphate-citrate buffer (pH 4.5) containing 0.015% hydrogen peroxide was added to the test tubes, 0.4 ml each, and incubated at room temperature for 15 minutes. Then, 2 ml of 1.5N aqueous sulfuric acid solution was added to the test tubes respectively to stop the enzymatic reactions. The absorbance of each solution was measured using a spectrophotometer at a wave-length of 450 nm.

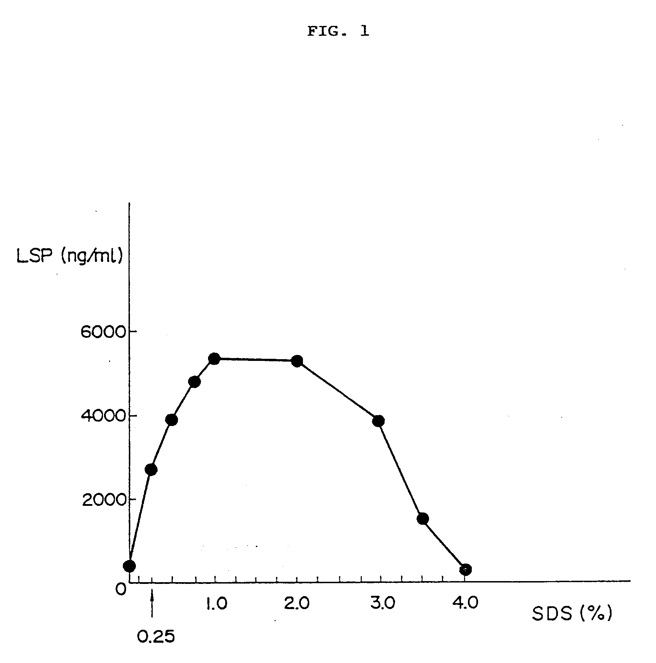

In the meantime, a calibration curve corresponding to individual concentrations of SDS was drawn using apoprotein as a standard substance, and the mixed solutions described above, as buffer solutions for dilution of amniotic fluid samples. The concentration of apoprotein (LSP) was determined from the absorbance at 450 nm wave-length using the calibration curve corresponding to SDS concentrations. The concentrations corresponding to individual SDS concentrations are shown in Figure 1.

As apparent from Figure 1, the antigen in the amniotic fluid samples (LSP) is recognized by the antibody relatively well in the range of SDS concentrations from 0.25 to 3.0 % in the presence of 2% Triton.

### Example 2

### Quantitation of pulmonary surfactant in amniotic fluid samples by change in concentration of Triton® buffer (immunoreaction: 37°C)

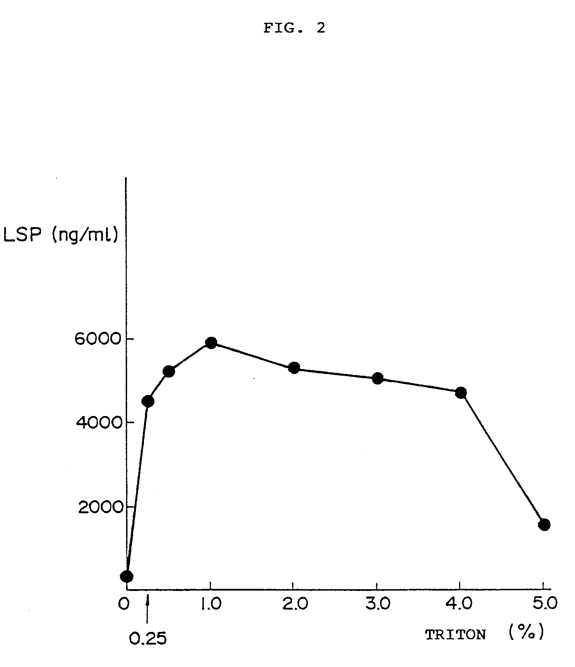

The same experiment as in Example 1 (3) was made except that mixed solutions of 1% SDS and Triton® X-100 buffer of varied concentrations (0, 0.25, 0.5, 1.0, 2.0, 3.0, 4.0 and 5.0%) were used for sample dilution. The relationship between the concentrations of Triton® and LSP values is shown in Figure 2. As apparent from Figure 2, the antigen in the amniotic fluid samples can be assayed relatively well in the range of Triton concentrations from 0.25 to 4.0% in the presence of 1% SDS.

### Example 3

### Quantitation of pulmonary surfactant in amniotic fluid samples by change of SDS concentrations (immunoreaction: 45°C)

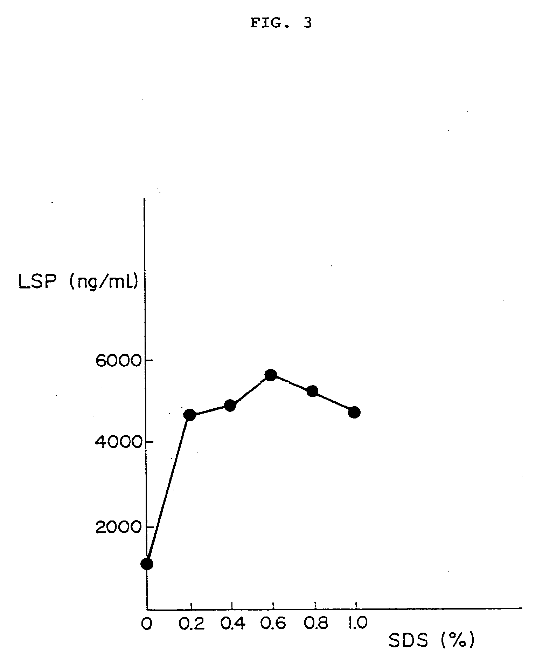

The same experiment as in Example 1 (3) was made except that mixed solutions of 2% Triton® X-100 buffer and SDS solutions of varied concentrations (0, 0.2, 0.4, 0.6, 0.8, 1.0%) were used for sample dilution and the immunoreactions were carried out at 45°C for 30 minutes. The relationship between SDS concentrations and LSP values is given in Figure 3. It is evident from Figure 3 that good results were obtained at 45°C in the range of SDS concentrations from 0.2 to 1.0% in the presence of 2% Triton and 0.6% SDS attained the maximum immunoreaction.

### Example 4

### The influence of immunoreaction time on the observed values of pulmonary surfactants in amniotic fluid samples (immunoreaction temperature : 45°C)

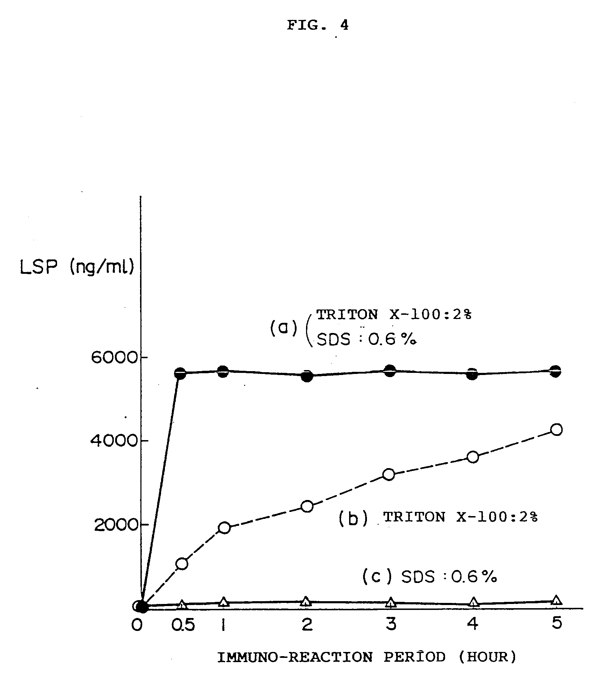

The standard substance (apoprotein), three kinds of buffer solutions for diluting amniotic fluid samples, (a) a mixture of 2% Triton® X-100 and 0.6% SDS, (b) 2% Triton® X-100 only, and (c) 0.6% SDS only, were used to conduct immunoreactions at 45°C varying the reaction time, 30 minutes, 1 hour, 2 hours, 3 hours, 4 hours and 5 hours, as in Example 1 (3). The concentrations of pulmonary surfactant in amniotic fluid were read from the calibration curves respectively at the prescribed times of immunoreaction and plotted on Figure 4.

Figure 4 shows that in (c) 0.6% SDS only the immunoreaction did not proceed at all and (b) 2% Triton® only retarded the reaction. On the contrary, (a) Triton®/SDS mixed solution allowed the observed values to reach the equilibrium point in 30 minutes and the effect of the mixed solution is definitely evident.

### Example 5

### Monoclonal antibody PC6 and labeled monoclonal antibody PE 10 which were prepared in Example 1 were used to determine pulmonary surfactant in sputum samples by changes of both SDS and Triton® concentrations.

Sputum was doubled in volume with physiological saline solution and homogenized. Solutions of 0, 0.5, 1.0, 1.5 and 2.0% SDS concentrations were individually combined with solutions of 0, 1, 2, 3 and 4% Triton® concentrations respectively to prepare 25 kinds of mixed solutions. Then, a sputum sample diluted with physiological saline solution was diluted 10 times with each of these 25 kinds of solutions.

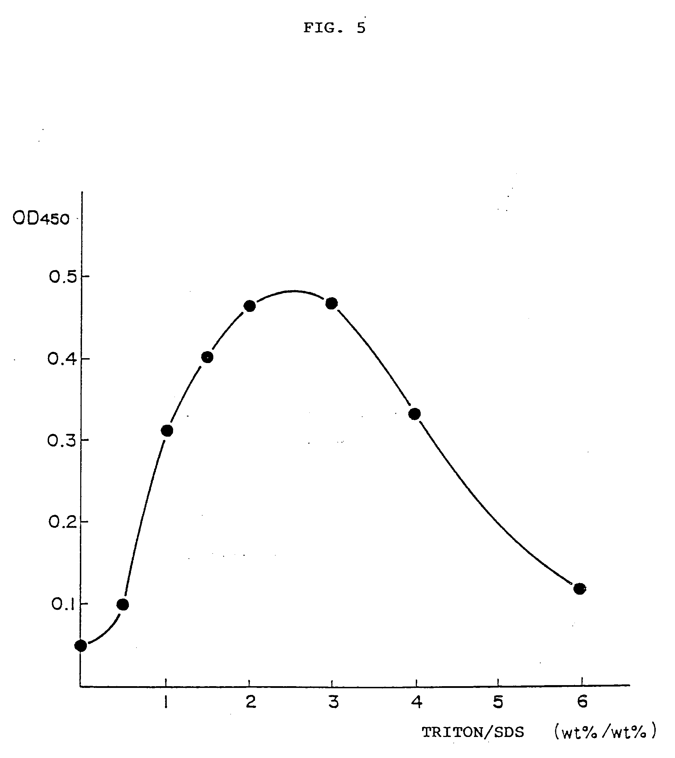

These mixtures containing the sample to be determined and a diluted solution containing monoclonal antibody PE 10 labeled with HRP were added, 200 µl each, to balls having monoclonal antibody PC6 immobilized thereon in test tubes to conduct immunoreactions at 37°C for 1 hour. Then, the solution was sucked off from the test tubes, the remainings were washed with physiological saline solution and combined with a 3 : 7 (V/V) mixture of methanol solution containing 1% 3,3'5,5'-tetramethylbenzidine and 1M phosphate-citrate buffer solution (pH 4.5) containing 0.015% hydrogen peroxide, 0.4 ml in each test tube. After incubation at room temperature for 15 minutes, 2 ml of 1.5N aqueous sulfuric acid was added to each test tube to stop the enzymatic reactions. Then, the absorbance of each solution was measured using a spectrophotometer at a wavelength of 450 nm. Absorbances corresponding to individual concentration ratios of Triton®/SDS are shown in Figure 5.

Figure 5 shows that the antigen in sputum (LSP) is well recognized by the antigen in the range of concentration ratios from 1.0 to 4.0 (w/w).

### Example 6

### Quantitation of pulmonary surfactant in sputum sampling by changes of SDS and Triton® concentrations using the latex agglutination method.

25 kinds of Triton®/SDS solution mixtures were mixed with an equal amount (50 µl) of sputum respectively so that the final concentrations are adjusted to SDS 0, 0.5, 1, 1.5, and 2% and Triton® 0, 1, 2, 3 and 4%. A latex emulsion bearing PC6 and PE10 on their surface, which recognize the apoprotein in pulmonary surfactant, (100 µl) was added to 100 µl of the Triton/SDS mixed solutions containing diluted sputums. After light stirring for 15 minutes, agglutination grades of the mixtures were recorded with symbols: -, ±, + and ++. The results are given in Table 1:

**Table 1**

| | | | | | | |
|---|---|---|---|---|---|---|
| Triton®/STD | 0 | 0.5 | 1.0 | 2.0 | 3.0 | 4.0 |
| | - | - | + | ++ | ++ | ± |

### Example 7

### Measurement of pulmonary surfactant in sputum

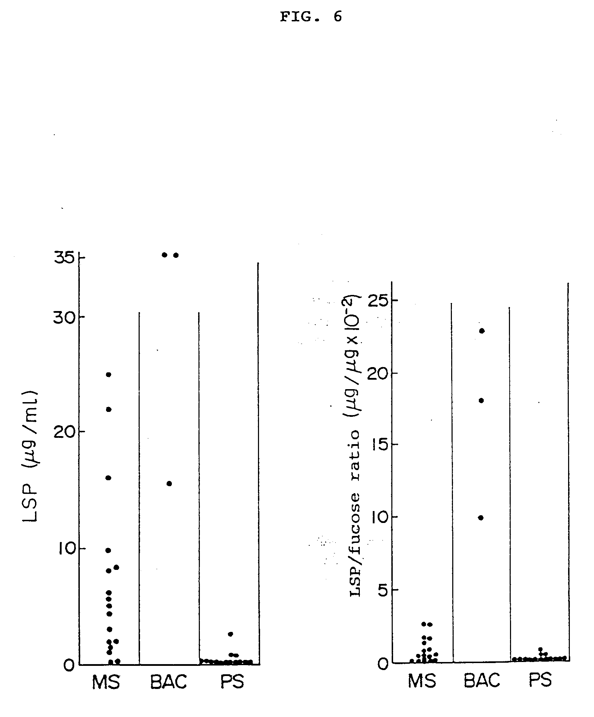

LSP/fucose concentration ratios were determined on individual samples from patients with membranous respiratory diseases (n = 17) and with bronchoalveolar epithelial cancer (n = 3) according to the conditions in Example 5 (Triton® 3.0% and SDS 1%) and the values classified into the two groups are given in Figure 6.

As apparent from Figure 6, the sputum from patients with bronchoalveolar epithelial cancer has higher LSP/fucose ratio than mucous sputum (MS) and puriform sputum (PS) from patients with chronic respiratory diseases. It evidently shows that the method according to the present invention can be used for the differential diagnosis.

In the figure, MS stands for mucous sputum and PS for puriform sputum from patients with chronic respiratory diseases, while BAC means the sputum from patients with bronchoalveolar epithelial cancer.

## Claims

1. An assay for quantitating human pulmonary surfactant by determining the amount of apoprotein in a sample of human pulmonary surfactant by the immunological method based on antigen-antibody reactions wherein the immunoreactions are conducted in a buffer solution containing a combination of nonionic surfactant and anionic surfactant, the ratio of said nonionic surfactant to said anionic surfactant being such that the antigen-antibody reaction is not inhibited by protein denaturation of the antigen protein and that the nonionic surfactant does not mask the effect of the anionic surfactant to disperse or loosen phospholipids and respiratory mucus surrounding the apoprotein.

2. An assay according to claim 1 wherein said apoprotein is determined using, as the antibodies, a first monoclonal antibody which recognizes the apoprotein of human pulmonary surfactant and a second labelled monoclonal antibody which also recognizes said apoprotein, but binds to a site on the antigen different from that to which the first antibody binds.

3. An assay according to claim 1 wherein said apoprotein is determined by conducting the immunoreaction using latex particles to which antibodies recognizing the apoprotein of human pulmonary surfactant are bonded.

4. An assay according to any of claims 1 to 3 wherein said anionic surfactant is an alkylsulfonate salt.

5. An assay according to any of claims 1 to 4 wherein said nonionic surfactant is a polyoxyethylene alkylphenol ether.

6. An assay according to claims 4 and 5 wherein the concentration ratio of nonionic surfactant to anionic surfactant is 1.0 to 4.0 by weight.

## Patentansprüche

1. Testverfahren zur Quantifizierung eines menschlichen Lungen-Surfactants durch Bestimmung der Menge eines Apoproteins in einer Probe des menschlichen Lungen-Surfactants durch immunologische Verfahren, die auf Antigen-Antikörper-Reaktionen basieren, worin die Immunreaktionen in einer Pufferlösung durchgeführt werden, die eine Kombination eines nichtionischen oberflächenaktiven Mittels und eines anionischen oberflächenaktiven Mittels enthält, wobei das Verhältnis des nichtionischen oberflächenaktiven Mittels zu dem anionischen oberflächenaktiven Mittel derart ist, daß die Antigen-Antikörper-Reaktion nicht durch Proteindenaturierrnng des Antigenproteins inhibiert wird und das nichtionische oberflächenaktive Mittel die Wirkung des anionischen oberflächenaktiven Mittels zum Dispergieren oder Ablösen der Phosphorlipide und den das Apoprotein umgebenden Atmungsschleim unwirksam macht.

2. Testverfahren nach Anspruch 1, worin das Apoprotein bestimmt wird unter Verwendung als Antikörper eines ersten monoklonalen Antikörpers, der das Apoprotein des menschlichen Lungen-Surfactants erkennt und eines zweiten, markierten monoklonalen Antikörpers, der ebenfalls das Apoprotein erkennt, jedoch an eine Stelle an das Antigen bindet, die von der an die der erste Antikörper bindet verschieden ist.

3. Testverfahren nach Anspruch 1, worin das Apoprotein durch Durchführung der Immunreaktion unter Verwendung von Latexteilchen bestimmt wird, an die die Antikörper gebunden werden, die das Apoprotein des menschlichen Lungen-Surfactants erkennen.

4. Testverfahren nach einem der Ansprüche 1 bis 3, worin das anionische oberflächenaktive Mittel ein Alkylsulfonatsalz ist.

5. Testverfahren nach einem der Ansprüche 1 bis 4, worin das nichtionische oberflächenaktive Mittel ein Polyoxyethylenalkylphenolether ist.

6. Testverfahren nach den Ansprüchen 4 und 5, worin das Konzentrationsverhältnis des nichtionischen oberflächenaktiven Mittels zu anionischem oberflächenaktiven Mittel 1,0 bis 4,0, bezogen auf das Gewicht, beträgt.

## Revendications

1. Procédé d'analyse pour évaluer la quantité d'agent tensio-actif pulmonaire humain en déterminant la quantité d'apoprotéine dans un échantillon d'agent tensio-actif pulmonaire humain par le procédé immunologique fondé sur les réactions antigène-anticorps, dans lequel les immunoréactions sont conduites dans une solution tampon contenant une combinaison d'agent tensio-actif non ionique et d'agent tensio-actif anionique, le rapport dudit agent tensio-actif non ionique audit agent tensio-actif anionique étant tel que la réaction antigène-anticorps n'est pas inhibée par la dénaturation protéique de la protéine antigénique et que l'agent tensio-actif non ionique ne masque pas l'effet de l'agent tensio-actif anionique pour disperser ou desserrer les phospholipides et le mucus respiratoire entourant l'apoprotéine.

2. Procédé d'analyse selon la revendication 1 dans lequel on détermine ladite apoprotéine en utilisant, comme anticorps, un premier anticorps monoclonal qui reconnaît l'apoprotéine de l'agent tensio-actif pulmonaire humain, et un second anticorps monoclonal marqué qui reconnaît également ladite apoprotéine, mais se lie sur l'antigène à un site qui diffère de celui auquel se lie le premier anticorps.

3. Procédé d'analyse selon la revendication 1 dans lequel on détermine ladite appoprotéine en conduisant l'immunoréaction en utilisant des particules de latex auxquelles sont liés des anticorps reconnaissant l'apoprotéine de l'agent tensio-actif pulmonaire humain.

4. Procédé d'analyse selon l'une quelconque des revendications 1 à 3 dans lequel ledit agent tensio-actif anionique est un sel d'alkylsulfonate.

5. Procédé d'analyse selon l'une quelconque des revendications 1 à 4 dans lequel ledit agent tensioactif non ionique est un éther polyoxyéthylènique alkylphénol.

6. Procédé d'analyse selon les revendications 4 et 5 dans lequel le rapport de concentration de l'agent tensio-actif non ionique à l'agent tensio-actif anionique est de 1,0 à 4,0 en poids.
